# EUROPEAN PATENT SPECIFICATION

(11) **EP 3 743 137 B1**
(45) Date of publication and mention of the grant of the patent: **10.06.2026**
(21) Application number: 19704946.3
(22) Date of filing: 25.01.2019
(51) Int. Cl.: A61M 5/31, A61M 39/20

(54) **FLUSH SYRINGE WITH SHIELDED TIP**
SPÜLSPRITZE MIT UMHÜLLTER SPITZE
SERINGUE POUR RINCER AVEC POINTE PROTÉGÉE

(30) Priority: 26.01.2018 US 201862622458 P; 22.01.2019 US 201916253739
(43) Date of publication of application: 02.12.2020
(73) Proprietor: Becton, Dickinson and Company, Franklin Lakes, NJ 07417-1880 (US)
(72) Inventor: MAHMOODIAN, Roza, New York 10018 (US)
(74) Representative: dompatent
(86) International application number: PCT/US2019/015097
(87) International publication number: WO 2019/147903

(56) References cited:
- EP-A1- 2 862 587
- WO-A1-2016/059336
- KR-B1- 101 750 352
- US-A1- 2013 030 414
- US-B1- 9 463 310

## Description

### TECHICAL FIELD

The present disclosure generally relates to syringe assemblies, and particularly to syringe assemblies comprising a physical barrier to prevent contact of the syringe tip with the surrounding non-sterile environment. Embodiments of the present disclosure ensure adherence to aseptic techniques for use in flush procedures for vascular access devices (VAD's). Embodiments of the present disclosure are also directed to technology to reduce the risk for bloodstream infections (CRBSI) and intravenous (IV) line patency maintenance including capping technology, particularly for syringe assemblies for use in flush procedures.

### BACKGROUND

Vascular access devices (VADs) are commonly used therapeutic devices, which include peripheral catheters and central venous catheters. If not properly maintained or if exposed to a non-sterile environment, the VADs can become contaminated, sealed with blood clots or spread infection. To ensure VADs are used properly and do not become sealed or infected, protocols to ensure sterile practice have been developed. These protocols include sterilizing the VAD and flushing the catheter with a flush solution. Catheters are flushed using syringe assemblies filled with various fluids. In some cases, different fluids are injected sequentially in accordance with the protocol. For example, a saline solution followed by an anticoagulant such as heparin. The size of the syringe used to flush intravenous (I.V.) lines varies by various factors including the size and length of the catheter. Typically syringes of 1ml, 3ml, 5ml and 10ml volume are used. VAD protocols usually recommend flush procedures be performed after catheter placement, before fluid infusion, and before and after drug administration, blood sampling, transfusions and parenteral nutrition. The goal of these flush procedures is to confirm catheter patency, avoid drug incompatibilities, ensure the complete drug dose administration, prevent thrombus formation and minimize the risk of blood stream infections.

Conventional flush syringes have a barrel with a luer tip at one end which is exposed to the non-sterile environment once the syringe tip is removed from packaging thus providing an opportunity for undesired contamination. Consequently, there is a need for a syringe, particularly a flush syringe, providing a physical barrier around the syringe tip which promotes aseptic practice by reducing or eliminating "touch" contamination of the syringe, particularly the tip of a syringe, with the surrounding non-sterile environment. EP 2 862 587 A1 discloses such a syringe according to the state of the art.

### SUMMARY

One aspect of die present disclosure pertains to a flush syringe assembly including a barrel having a side wall with an inside surface defining a chamber for retaining a fluid, an open proximal end, a distal end including a distal wall with an elongate tip extending distally therefrom having a passageway therethrough in fluid communication with said chamber. The flush syringe assembly also includes a collar mounted on the distal wall of the barrel and surrounding the elongate tip. The collar includes at least one side wall having an inside surface defining a compartment, an open distal end, a proximal end adjacent the distal wall of the barrel. The flush syringe assembly also includes an elongated plunger rod disposed within the barrel, the plunger rod comprising a distal portion and a proximal portion. The plunger rod also includes a distal end including a stopper slidably positioned in fluid-tight engagement with the inside surface of the barrel for drawing fluid into and driving fluid out of the chamber by movement of the stopper relative to the barrel. The elongated plunger rod extends outwardly from the open proximal end of the barrel.

In one or more embodiments, the distal wall of the barrel includes a plurality of threads to connect the barrel to the collar. In certain embodiments, the proximal end of the collar may be threaded onto the distal wall of the barrel.

According to the invention, the compartment of the collar surrounds the elongated tip. In one or more embodiments, the collar may be removable.

In one or more embodiments, die collar may have a convex inner surface or a concave inner surface.

In one or more embodiments, the collar has a shape of a trapezoidal prism.

In one or more embodiments, the fluid is a flush fluid.

According to the invention, the flush syringe assembly also comprises a removable cap having a body, a proximal end, and a closed distal end may be mounted on the distal end of the collar.

In one or more embodiments, the distal wall of the collar may include a plurality of threads to connect the collar to the removable cap.

In one or more embodiments, the collar may be removable.

In one or more embodiments, the removable cap may include an outward protrusion extending from the body of the cap and corresponding with the opening of the distal end of die elongate tip.

In one or more embodiments, the removable cap has a cross-sectional shape that is triangular, square, pentagonal, hexagonal, heptagonal, octagonal, symmetric or non-symmetric polygonal.

### BRIEF DESCRIPTION OF THE DRAWINGS

FIG. 1 illustrates a partial perspective view of a flush syringe with a collar in accordance with one or more embodiments of the present disclosure;
FIG. 2 illustrates a top perspective view of a flush syringe with a collar in accordance with one or more embodiments of the present disclosure;
FIG. 3 illustrates a side view of a flush syringe with a collar and cap in accordance with one or more embodiments of the present disclosure;
FIG. 4 illustrates a cross-sectional perspective view of a flush syringe with a collar and a cap in accordance with one or more embodiments of the present disclosure;
FIG. 5 illustrates a top perspective view of a flush syringe with a collar in accordance with one or more embodiments of die present disclosure;
FIG. 6 illustrates a side view of a flush syringe with a collar and cap in accordance with one or more embodiments of the present disclosure;
FIG. 7 illustrates a cross-sectional perspective view of a flush syringe with a collar and a cap in accordance with one or more embodiments of the present disclosure;
FIG. 8 illustrates a perspective view of an assembled flush syringe with a collar and a cap in accordance with one or more embodiments of the present disclosure; and
FIG. 9 illustrates a perspective view of a flush syringe with a collar and a cap that is removed in accordance with one or more embodiments of the present disclosure.

### DETAILED DESCRIPTION

Before describing several exemplary embodiments of the present disclosure, it is to be understood that the disclosure is not limited to the details of construction or process steps set forth in the following description. The disclosure is capable of other embodiments and of being practiced or being carried out in various ways.

With respect to terms used in this disclosure, the following definitions are provided.

Reference to "flush syringe assembly" includes syringes that are indicated for use in the flushing of VADs. The practice of flushing ensures and maintains catheter patency and helps prevent the mixing of incompatible pharmaceuticals.

As used herein, the use of "a," "an," and "the" includes the singular and plural.

As used herein, the term "catheter related bloodstream infection" or "CRBSI" refers to any infection resulting from the presence of a catheter or IV line.

As used herein, the term "Luer connector" refers to a connection collar that is the standard way of attaching syringes, catheters, hubbed needles, IV tubes, etc. to each other. The Luer connector consists of male and female interlocking tubes, slightly tapered to hold together better with even just a simple pressure/twist fit. Luer connectors can optionally include an additional outer rim of threading, allowing them to be more secure. The Luer connector male end is generally associated with a flush syringe and can interlock and connect to the female end located on the VAD. A Luer connector comprises a distal end, a proximal end, an irregularly shaped outer wall, a profiled center passageway for fluid communication from the chamber of the barrel of a syringe to the hub of a VAD. A Luer connector also has a distal end channel that releasably attaches die Luer connector to the hub of a VAD, and a proximal end channel that releasably attaches the Luer connector to the barrel of a syringe.

Because clinicians have to handle multiple components while flushing intravenous (IV) catheters, a certain level of dexterity is required to carry out the procedure while preventing the syringe tip from coming into contact with the surrounding environment. If the syringe tip touches any non-sterile surfaces, "touch" contamination can occur which can cause microbial growth in the IV line and consequently lead to incidents of catheter-associated-bloodstream infection ("CRBSI") and central line-associated bloodstream infection ("CLABSI") which are very costly and lethal. Embodiments of the present disclosure relate to a flush syringe having a collar surrounding the elongate tip of the syringe. The collar provides a physical barrier around the syringe tip. Because of its shape, the collar of the present disclosure can help facilitate the alignment of the flush syringe with the catheter hub and/or needle-free connector and thus reduce the chances of "touch" contamination.

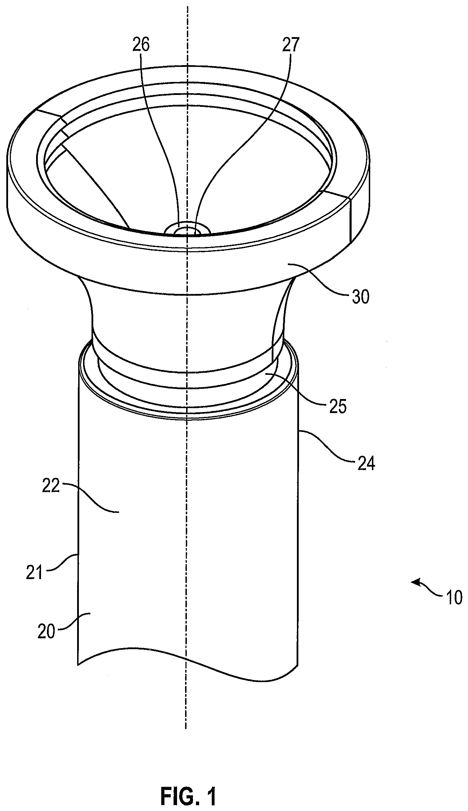

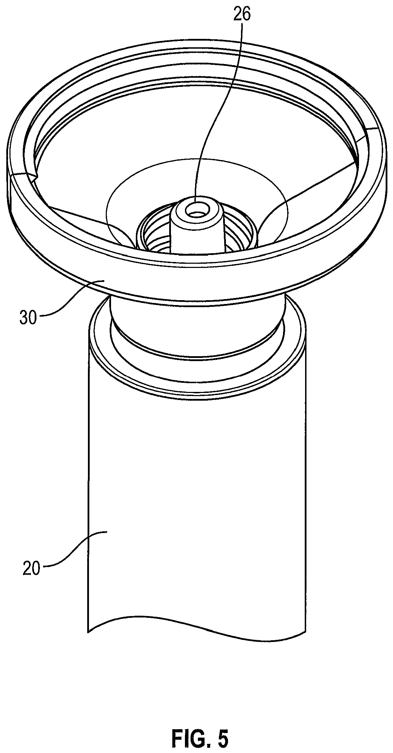

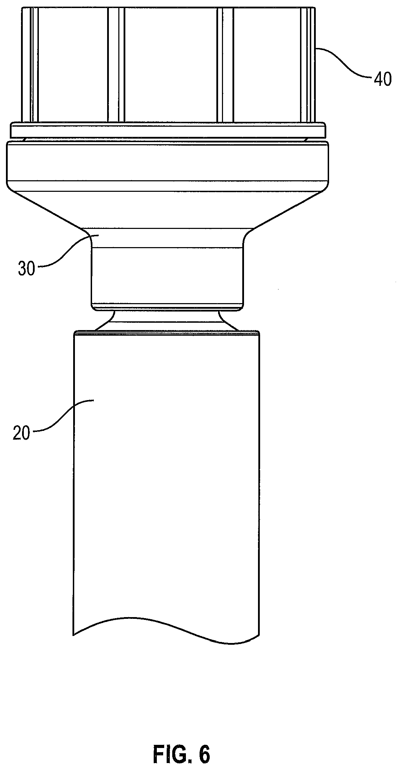

Provided are syringe assemblies that include a plunger rod and a syringe barrel having an open proximal end and a distal tip, the distal syringe tip surrounded by a collar to facilitate alignment of the syringe with a catheter hub or needle-free connector, as well as, reducing contamination of the syringe by preventing contact of the syringe tip with the surrounding non-sterile environment. Referring to Figures 1-7, a syringe assembly 10 according to the present disclosure generally comprises a barrel 20, including a side wall 21 having an inside surface defining a chamber 22 for retaining a fluid. In one or more embodiments, the fluid is a flush fluid. The barrel 20 further includes an open proximal end (not shown) and a distal end 24 having a distal wall 25 and an elongated tip 26 extending distally therefrom, the elongated tip 26 having a passageway 27 therethrough in fluid communication with the chamber, the distal wall 25 adapted for connection to a collar 30. The open proximal end of the barrel is disposed on the opposite end of the distal end 24. The distal wall 25 may comprise a plurality of threads 28 for attachment to a vascular access device, however the vascular access device may engage onto the distal wall 25 via a variety of methods, including press-fitting, adhering, detenting, co-injection molding, etc.

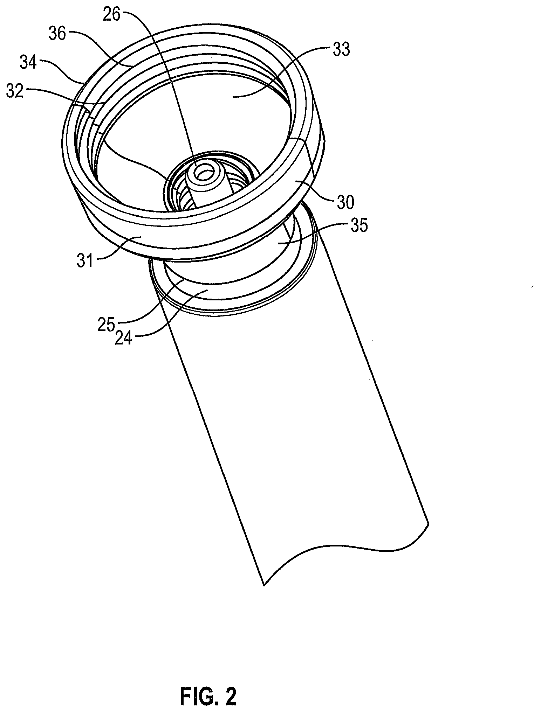

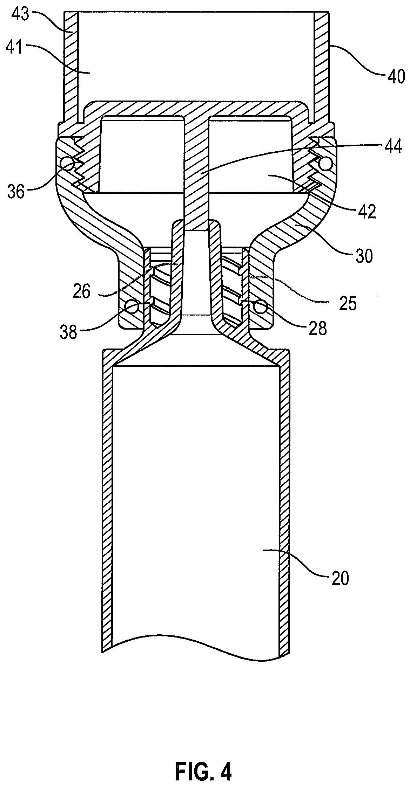

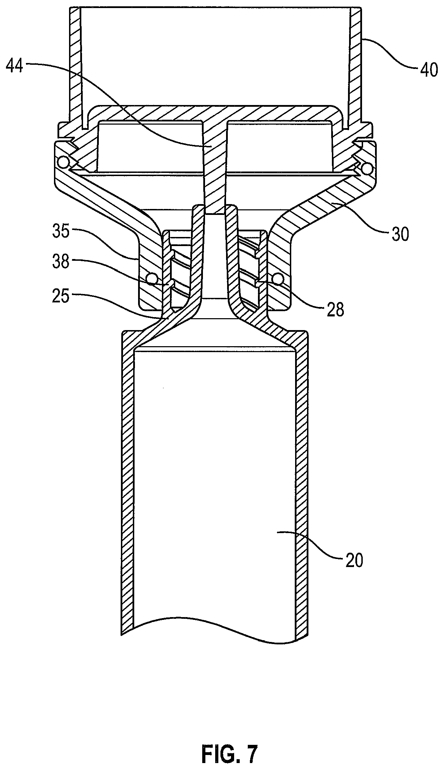

As shown in Fig. 2, collar 30 is mounted on the distal wall 25 of the barrel, the collar 30 including at least one side wall 31 having an inside surface 32 defining a compartment 33 surrounding the elongated tip 26, an open distal end 34, and a proximal end 35 adjacent and in contact with the distal wall 25 of the barrel. Elongated tip 26 adapted for connection to a hub of a vascular access device. The collar 30 may comprise a plurality of threads 36 on the inside surface for connection to a removable cap 40. As shown in Figs. 4 and 7, the collar 30 may comprise an engagement portion 38 on the proximal end 35 for attachment to the distal wall 25 of the barrel. Upon manufacture, the flush syringe assembly 10 can be provided with the collar 30 connected at the proximal end 35 onto the distal wall 25 of the barrel. The engagement may be via a variety of methods not limited to: threading, press-fitting, adhering, detenting, co-injection molding, etc. The shape of the collar 30 can vary. Collar 30 may have shapes including, but not limited to, a convex inner surface (for example a paraboloid), concave inner surface, with a straight profile (i.e., semi conical shape), or have the shape of a trapezoidal prism. The length of this extension from the main body of syringe and the degree of openness/straightness of the profile (how wide the collar is at the end farthest from the syringe barrel) can vary.

In one or more embodiments, the collar 30 may be removable from the syringe assembly 10.

In an embodiment, the collar 30 may comprise a plurality of threads adapted for connection to the hub of the vascular access device. In one or more embodiments, the collar 30 surrounds an elongate tip adapted for connection to the hub of the vascular access devices. In one or more embodiments, the elongate tip is a Luer tip.

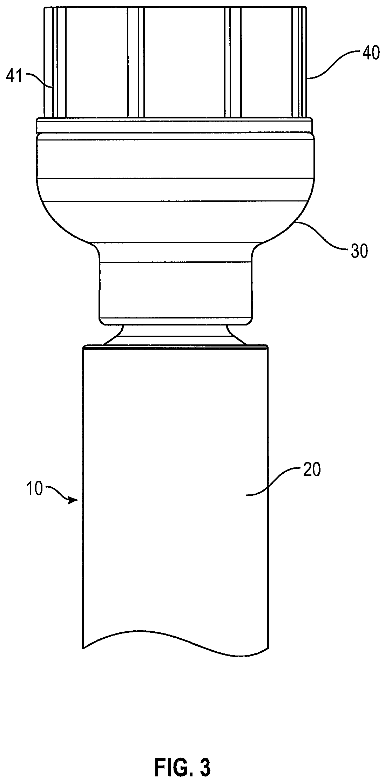

As shown in Figures 3, 4 and 6, the open distal end 34 of the collar 30 may comprise a removable cap 40. The removable cap 40 includes a body 41 with a proximal end 42, a closed distal end 43. The distal end 43 and the proximal end 42 define the length of the cap 40. FIGS. 3, 4, 6 and 7 show a side view and a cross-sectional view, respectively, of a cap 40 in accordance with one or more embodiments of the present disclosure. It should be noted that cap distal wall 430 may be recessed within the cap distal end 43, such that a cap recess 431 at cap distal end 43 is formed. Upon assembly, the proximal end 42 of the cap 40 is adjacent the distal end 34 of the collar 30. As shown in Figs. 4 and 7, cap 40 may comprises an outward protrusion 44 that extends from the body 41 of the cap 40 and corresponds with the opening of the distal end of the elongate tip 26. The proximal end 42 of removable cap 40 may comprise a plurality of threads on the outside surface of the cap for attachment to corresponding grooves disposed on the inside surface of the distal end of the collar 30. In one or more embodiments, at least one screw thread adapted to allow the cap to be screwed onto collar 30.

The cross-sectional shape of the cap 40 can he any suitable shape including, but not limited to, triangular, square, pentagonal, hexagonal, heptagonal, octagonal, symmetric or non-symmetric polygonal. The shape of the cap 40 can provide a comfortable feel for the user and enhanced gripping ability to allow the user to easily connect or disconnect the cap from the collar 30. In some embodiments, the cap 40 is irregularly shaped. As used in this specification and the appended claims, the term "irregularly shaped" means that the cross-sectional shape provides a surface or edge that is detrimental to free rotation about the cross-section. For example, a hexagon or oval shape would be considered "irregular".

The syringe assembly 10 may be filled with flush solution using known methods. Additionally, the syringe assembly 10 may be provided pre-filled from the manufacturer or supplier. The flush solution may be any solution intended for flushing or maintaining performance of VAD's. It is preferred that the flush solution be selected from the group consisting of saline flush solution and heparin lock flush solution. These solutions are known in the art and are readily available. An example of a saline flush solution includes, but is not limited to, 0.9% sodium chloride USP for injection. An example of a heparin lock flush solution includes but is not limited to 0.9% sodium chloride with 100 USP units of heparin sodium per mL or 10 USP units of heparin sodium per mL.

Once the connection of the syringe assembly 10 to the VAD is completed, fluid communication from the barrel 20 of the syringe to the vascular access device can occur. Fluid is drawn from the barrel 20 through the integral passageway 27 into the IV or catheter. Because of the presence of the collar 30, fluid communication through a vascular access device and into a patient is conducted under aseptic conditions without any additional swabbing steps and diligence on the part of the clinician.

In one or more embodiments, the collar 30 can be integrally formed on the distal wall 25 of the syringe barrel 20 for fluid communication to the vascular access device.

The barrel may also include a tip which extends distally from the barrel. The tip can have an outer diameter that is different from or the same as the outer diameter of the rest of the barrel. For example, as shown in the Figures, the outer diameter of the tip has a smaller outer diameter than the barrel portion that is proximal of the tip. The tip of the barrel may include a luer slip connection or a locking luer type collar concentrically surrounding the tip or within the tip.

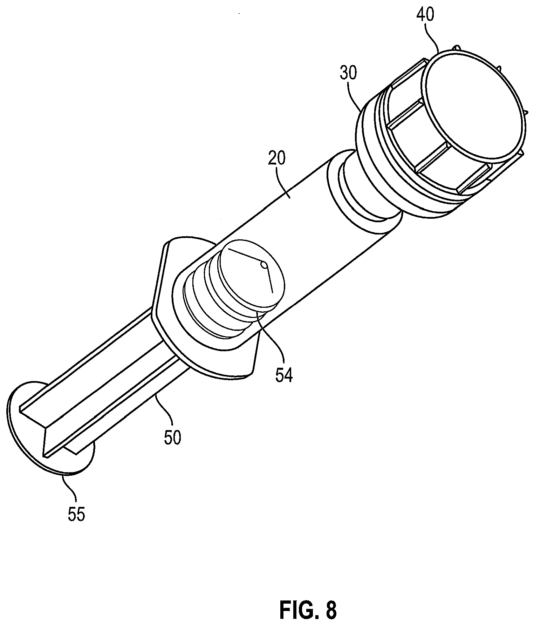

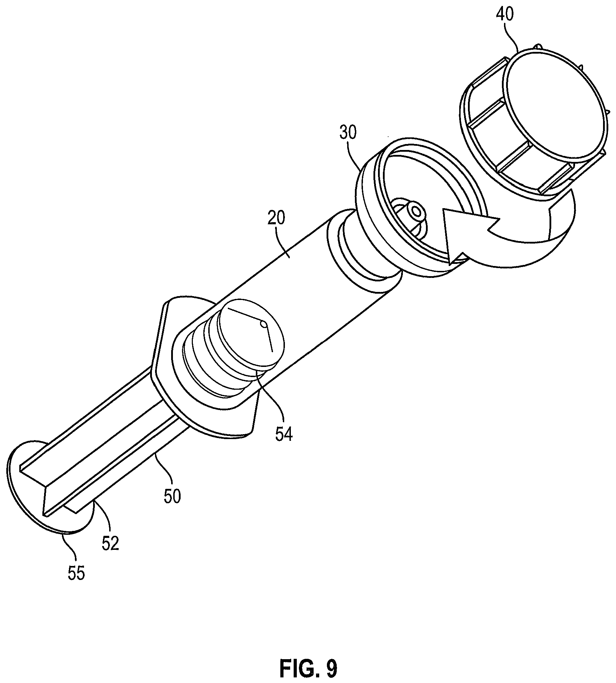

FIG. 8 illustrates a perspective view of an assembled flush syringe with a collar and a cap in accordance with one or more embodiments of the present disclosure. FIG. 9 illustrates a perspective view of a flush syringe with a collar and a cap that is removed in accordance with one or more embodiments of the present disclosure.

An elongated plunger rod 50 may include a distal portion and a proximal portion 52, the plunger rod further comprising a distal end 51 including a stopper 54 slidably positioned in fluid-tight engagement with the inside surface of the barrel for drawing fluid into and driving fluid out of the chamber by movement of the stopper relative to the barrel, the elongated plunger rod extending outwardly from the open proximal end 23 of the barrel, the stopper having a distal surface.

An elongate plunger rod may be disposed within the barrel 20. The plunger rod includes an elongate body portion with a proximal end and a distal end.

The elongate body portion of the plunger rod has an axial length extending from the proximal end to the distal end. The body portion may include a single beam or features, which may have cylindrical or other shapes. The body portion may be formed by two perpendicularly intersecting beams.

The plunger rod 50 may also include a thumb press 55 at the proximal end 52 of the elongate body portion. The shape of the thumbpress can vary depending on the desired usage of the flush syringe assembly. The shape of the thumb press may be round, square, rectangular, triangular, oval, pentagonal, hexagonal. and cruciform.

A stopper 54 can be connected to the distal end of the plunger rod. The shape and size of the stopper can be any suitable shape or size depending on, for example, the shape and size of the barrel and plunger rod. The plunger rod 50 is slidably positioned in the barrel 20 so that the stopper is in fluid-tight contact with the inside surface of the barrel and so that distal movement of the plunger rod 50 relative to the barrel 20 causes the stopper 54 to push the fluid out of the barrel. In some embodiments, the stopper is slidably positioned in fluid-tight contact with the inside surface of the barrel for driving fluid out of the chamber by movement of the stopper relative to the barrel. The stopper can be connected to the distal end of the elongate plunger rod by any suitable means. In some embodiments, the stopper is connected by a mechanical connection such as interaction of complementary screw threads and press-fit connections. The stopper may be slidably positioned in fluid-tight engagement with the inside surface of the barrel for drawing fluid into and driving fluid out of the chamber.

The stopper may be made of any material suitable for providing a seal with the inside surface of the barrel. For example, the stopper may be made of thermoplastic elastomers, natural rubber, synthetic rubber or thermoplastic materials and combinations thereof. The stopper may be integrally formed or composed of separate components of die same or different materials joined together. The plunger rod may be made of material which is more rigid than the stopper such as polypropylene, polyethylene and the like. Materials should be chosen to be compatible with the procedure being used.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the scope of the present disclosure as disclosed.

## Claims

1. A flush syringe assembly comprising:
a barrel (20) including a side wall (21) having an inside surface defining a chamber (22) for retaining a fluid, an open proximal end (23) and a distal end (24) including a distal wall (25) with an elongate tip (26) extending distally therefrom having a passageway (27) therethrough in fluid communication with said chamber (22);
an elongated plunger rod disposed within the barrel (20), the plunger rod comprising a distal end and a proximal end, the distal end (24) including a stopper slidably positioned in fluid-tight engagement with the inside surface of the barrel (20) for drawing fluid into and driving fluid out of the chamber (22) by movement of the stopper relative to the barrel (20), the elongated plunger rod extending outwardly from the open proximal end (23) of the barrel (20), the stopper having a distal surface, wherein
the distal wall (25) and the elongate tip (26) extend distally from the distal end (24), the elongate tip (26) having a passageway therethrough in fluid communication with said chamber (22);
a collar (30) mounted on and in contact with the distal wall (25) of the barrel (20), the collar (30) axially extends farther from the barrel (20) than completely surrounding the elongate tip (26) of the barrel (20), the collar (30) including at least one continuous side wall (31) having an inside surface (32) defining a compartment (33), an open distal end (34) extending farther than past and completely surrounding the distal end of the elongate tip, a proximal end (35) adjacent the distal wall (25) of the barrel (20), the inner diameter and the outer diameter of the distal end of the collar is larger than the inner diameter and the outer diameter of the proximal end of the collar providing a physical barrier around the elongate tip to reduce or eliminate contamination of the elongate tip from the surrounding non-sterile environment; and a removable cap (40) having a body, a proximal end (42), and a closed distal end (43), the removable cap (40) being mounted on the distal end (24) of the collar (30).

2. The flush syringe assembly of claim 1, wherein the distal wall (25) of the barrel (20) includes an engagement portion to connect the barrel (20) to the collar (30).

3. The flush syringe assembly of claim 2, wherein the proximal end (35) of the collar (30) is threaded onto the distal wall (25) of the barrel (20).

4. The flush syringe assembly of claim 1, wherein the compartment (33) of the collar (30) surrounds the elongated tip (26).

5. The flush syringe assembly of claim 1, wherein the collar (30) is removable.

6. The flush syringe assembly of claim 1, wherein the collar (30) has a convex inner surface.

7. The flush syringe assembly of claim 1, wherein the collar (30) has a concave inner surface.

8. The flush syringe assembly of claim 1, wherein the collar (30) has a shape of a trapezoidal prism.

9. The flush syringe assembly of claim 1, wherein the fluid is a flush fluid.

10. The flush syringe assembly of claim 1, wherein the distal wall (25) of the barrel (20) includes a plurality of threads (28) to connect the barrel (20) to the collar (30).

11. The flush syringe assembly of claim 1 wherein the distal wall of the collar (30) includes a plurality of threads to connect the collar (30) to the removable cap (40).

12. The flush syringe assembly of claim 1, wherein the removable cap (40) includes an outward protrusion (44) extending from the body of the removable cap (40) and corresponding with the passageway (27) on the distal end (24) of the elongated tip (26).

13. The flush syringe assembly of claim 1, wherein the removable cap (40) has a cross-sectional shape that is triangular, square, pentagonal, hexagonal, heptagonal, octagonal, symmetric or non-symmetric polygonal.

## Patentansprüche

1. Spülspritzenbaugruppe, die aufweist:
einen Zylinder (20), der aufweist: eine Seitenwand (21) mit einer Innenfläche, die eine Kammer (22) zum Halten eines Fluids definiert, ein offenes proximales Ende (23), und ein distales Ende (24), das eine distale Wand (25) aufweist mit einer sich distal von dieser erstreckenden längliche Spitze (26), die einen durch diese verlaufenden Durchlass (27) in Fluidverbindung mit der Kammer (22) aufweist;
eine in dem Zylinder (20) angeordnete längliche Kolbenstange, wobei die Kolbenstange ein distales Ende und ein proximales Ende aufweist, wobei das distale Ende (24) einen Stopfen aufweist, der gleitend verschiebbar in fluiddichtem Eingriff mit der Innenfläche des Zylinders (20) positioniert ist, um durch Bewegen des Stopfens in Bezug auf den Zylinder (20) Fluid in die Kammer (22) zu ziehen und aus derselben zu treiben, wobei sich die längliche Kolbenstange von dem offenen proximalen Ende (23) des Zylinders (20) nach außen erstreckt, wobei der Stopfen eine distale Fläche aufweist, wobei
die distale Wand (25) und die längliche Spitze (26) sich distal von dem distalen Ende (24) erstrecken, wobei die längliche Spitze (26) einen durch diese verlaufenden Durchlass aufweist, der in Fluidverbindung mit der Kammer (22) steht;
eine Manschette (30), die an der distalen Wand (25) des Zylinders (20) angebracht ist und mit dieser in Kontakt steht, wobei sich die Manschette (30) axial weiter von dem Zylinder (20) erstreckt als die längliche Spitze (26) des Zylinders (20) vollständig umgibt, wobei die Manschette (30) mindestens eine durchgehende Seitenwand (31) mit einer Innenfläche (32), die eine Kammer (33) definiert, ein offenes distales Ende (34), das sich weiter als über das distale Ende der länglichen Spitze hinaus erstreckt und dieses vollständig umgibt, und ein proximales Ende (35) aufweist, das an die distale Wand (25) des Zylinders (20) angrenzt, wobei der Innendurchmesser und der Außendurchmesser des distalen Endes der Manschette größer sind als der Innendurchmesser und der Außendurchmesser des proximalen Endes der Manschette, wodurch eine physische Barriere um die längliche Spitze herum geschaffen wird, um eine Kontamination der länglichen Spitze durch die umgebende unsterile Umgebung zu verringern oder zu beseitigen; und
eine abnehmbare Kappe (40) mit einem Körper, einem proximalen Ende (42) und einem geschlossenen distalen Ende (43), wobei die abnehmbare Kappe (40) auf dem distalen Ende (24) der Manschette (30) angebracht ist.

2. Spülspritzenbaugruppe nach Anspruch 1, wobei die distale Wand (25) des Zylinders (20) einen Eingriffsabschnitt zur Verbindung des Zylinders (20) mit der Manschette (30) aufweist.

3. Spülspritzenbaugruppe nach Anspruch 2, wobei das proximale Ende (35) der Manschette (30) auf die distale Wand (25) des Zylinders (20) geschraubt ist.

4. Spülspritzenbaugruppe nach Anspruch 1, wobei die Kammer (33) der Manschette (30) die längliche Spitze (26) umgibt.

5. Spülspritzenbaugruppe nach Anspruch 1, wobei die Manschette (30) abnehmbar ist.

6. Spülspritzenbaugruppe nach Anspruch 1, wobei die Manschette (30) eine konvexe Innenfläche aufweist.

7. Spülspritzenbaugruppe nach Anspruch 1, wobei die Manschette (30) eine konkave Innenfläche aufweist.

8. Spülspritzenbaugruppe nach Anspruch 1, wobei die Manschette (30) eine Form eines trapezförmigen Prismas hat.

9. Spülspritzenbaugruppe nach Anspruch 1, wobei das Fluid ein Spülfluid ist.

10. Spülspritzenbaugruppe nach Anspruch 1, wobei die distale Wand (25) des Zylinders (20) eine Vielzahl von Gewindegängen (28) aufweist, um den Zylinder (20) mit der Manschette (30) zu verbinden.

11. Spülspritzenbaugruppe nach Anspruch 1, wobei die distale Wand der Manschette (30) eine Vielzahl von Gewindegängen zum Verbinden der Manschette (30) mit der abnehmbaren Kappe (40) aufweist.

12. Spülspritzenbaugruppe nach Anspruch 1, wobei die abnehmbare Kappe (40) einen nach außen gerichteten Vorsprung (44) aufweist, der sich von dem Körper der abnehmbaren Kappe (40) erstreckt und mit dem Durchlass (27) am distalen Ende (24) der länglichen Spitze (26) korrespondiert.

13. Spülspritzenbaugruppe nach Anspruch 1, wobei die abnehmbare Kappe (40) eine dreieckige, quadratische, pentagonale, hexagonale, heptagonale, oktogonale, symmetrisch oder nicht symmetrisch polygonale Querschnittsform aufweist.

## Revendications

1. Ensemble seringue de rinçage comprenant :
un cylindre (20) comportant une paroi latérale (21) présentant une surface intérieure définissant une chambre (22) pour retenir un fluide, une extrémité proximale ouverte (23) et une extrémité distale (24) comportant une paroi distale (25) avec une pointe allongée (26) s'étendant distalement à partir de celle-ci comportant un passage (27) à travers celle-ci en communication fluidique avec ladite chambre (22) ;
une tige de piston allongée disposée à l'intérieur du cylindre (20), la tige de piston comprenant une extrémité distale et une extrémité proximale, l'extrémité distale (24) comportant un bouchon positionné de manière coulissante en prise étanche aux fluides avec la surface intérieure du cylindre (20) pour aspirer du fluide dans la chambre (22) et l'extraire de celle-ci par un mouvement du bouchon par rapport au cylindre (20), la tige de piston allongée s'étendant vers l'extérieur à partir de l'extrémité proximale ouverte (23) du cylindre (20), le bouchon présentant une surface distale, dans lequel la paroi distale (25) et la pointe allongée (26) s'étendent distalement à partir de l'extrémité distale (24), la pointe allongée (26) présentant un passage à travers celle-ci en communication fluidique avec ladite chambre (22) ;
un collier (30) monté sur la paroi distale (25) du cylindre (20) et en contact avec celle-ci, le collier (30) s'étend axialement plus loin du cylindre (20) qu'il n'entoure complètement la pointe allongée (26) du cylindre (20), le collier (30) comportant au moins une paroi latérale continue (31) comportant une surface intérieure (32) définissant un compartiment (33), une extrémité distale ouverte (34) s'étendant au-delà de l'extrémité distale de la pointe allongée et l'entourant complètement, une extrémité proximale (35) adjacente à la paroi distale (25) du cylindre (20), le diamètre interne et le diamètre externe de l'extrémité distale du collier sont supérieurs au diamètre interne et au diamètre externe de l'extrémité proximale du collier fournissant une barrière physique autour de la pointe allongée pour réduire ou éliminer la contamination de la pointe allongée par l'environnement non stérile environnant ; et un capuchon amovible (40) comportant un corps, une extrémité proximale (42) et une extrémité distale fermée (43), le capuchon amovible (40) étant monté sur l'extrémité distale (24) du collier (30).

2. Ensemble seringue de rinçage selon la revendication 1, dans lequel la paroi distale (25) du cylindre (20) comporte une partie de mise en prise pour relier le cylindre (20) au collier (30).

3. Ensemble seringue de rinçage selon la revendication 2, dans lequel l'extrémité proximale (35) du collier (30) est vissée sur la paroi distale (25) du cylindre (20).

4. Ensemble seringue de rinçage selon la revendication 1, dans lequel le compartiment (33) du collier (30) entoure la pointe allongée (26).

5. Ensemble seringue de rinçage selon la revendication 1, dans lequel le collier (30) est amovible.

6. Ensemble seringue de rinçage selon la revendication 1, dans lequel le collier (30) présente une surface interne convexe.

7. Ensemble seringue de rinçage selon la revendication 1, dans lequel le collier (30) présente une surface interne concave.

8. Ensemble seringue de rinçage selon la revendication 1, dans lequel le collier (30) présente une forme de prisme trapézoïdal.

9. Ensemble seringue de rinçage selon la revendication 1, dans lequel le fluide est un fluide de rinçage.

10. Ensemble seringue de rinçage selon la revendication 1, dans lequel la paroi distale (25) du cylindre (20) comporte une pluralité de filetages (28) pour relier le cylindre (20) au collier (30).

11. Ensemble seringue de rinçage selon la revendication 1, dans lequel la paroi distale du collier (30) comporte une pluralité de filetages pour relier le collier (30) au capuchon amovible (40).

12. Ensemble seringue de rinçage selon la revendication 1, dans lequel le capuchon amovible (40) comporte une saillie (44) vers l'extérieur s'étendant à partir du corps du capuchon amovible (40) et correspondant au passage (27) sur l'extrémité distale (24) de la pointe allongée (26).

13. Ensemble seringue de rinçage selon la revendication 1, dans lequel le capuchon amovible (40) présente une forme de section transversale qui est triangulaire, carrée, pentagonale, hexagonale, heptagonale, octogonale, symétrique ou polygonale non symétrique.
